# NEUE EUROPÄISCHE PATENTSCHRIFT

(11) **EP 1 740 163 B2**
(45) Veröffentlichungstag und Bekanntmachung des Hinweises auf die Entscheidung über den Einspruch: **15.01.2020**
(45) Hinweis auf die Patenterteilung: 14.07.2010
(21) Anmeldenummer: 05730867.8
(22) Anmeldetag: 15.04.2005
(51) Int. Cl.: A61K 31/00, A61K 31/56, A61K 31/57, A61P 15/18

(54) **MEHRPHASENPRÄPARAT ZUR KONTRAZEPTION AUF DER BASIS EINES NATÜRLICHEN ESTROGENS**
MULTI-PHASE CONTRACEPTIVE PREPARATION BASED ON A NATURAL ESTROGEN
PREPARATION CONTRACEPTIVE A PLUSIEURS PHASES A BASE D'UN OESTROGENE NATUREL

(30) Priorität: 20.04.2004 DE 102004019743
(43) Veröffentlichungstag der Anmeldung: 10.01.2007
(73) Patentinhaber: Bayer Intellectual Property GmbH, 40789 Monheim (DE)
(72) Erfinder: ENDRIKAT, Jan, Quebec H9H 5B5 (CA); DÜSTERBERG, Bernd, 16727 Oberkrämer (DE)
(74) Vertreter: Plougmann Vingtoft a/s
(86) Internationale Anmeldenummer: PCT/EP2005/004022
(87) Internationale Veröffentlichungsnummer: WO 2005/102247

(56) Entgegenhaltungen:
- EP-A- 0 770 388
- WO-A-2004/112797
- US-B1- 6 312 722
- US-B1- 6 670 350
- ZIMMERMANN H ET AL: "Toxicology of dienogest" DRUGS OF TODAY 1999 SPAIN, Bd. 35, Nr. SUPPL. C, 1999, Seiten 13-26, XP008054776 ISSN: 0025-7656
- GRASER T ET AL: "Dienogest as a progestin for hormone replacement therapy" DRUGS OF TODAY 1999 SPAIN, Bd. 35, Nr. SUPPL. C, 1999, Seiten 115-126, XP008054769 ISSN: 0025-7656
- MOORE C ET AL: "Influence of dienogest on ovulation in young fertile women" CLINICAL DRUG INVESTIGATION, Bd. 18, Nr. 4, Oktober 1999 (1999-10), Seiten 271-278, XP008054770 ISSN: 1173-2563
- OETTEL M ET AL: "THE PRECLINICAL AND CLINICAL PROFILE OF DIENOGEST. A SHORT OVERVIEW" DRUGS OF TODAY / MEDICAMENTOS DE ACTUALIDAD, J.R. PROUS SS.A. INTERNATIONAL PUBLISHERS, ES, Bd. 35, 1999, Seiten 3-12, XP000909647 ISSN: 0025-7656
- STRECKE J ET AL: "UNTERSUCHUNGEN ZUM VERHALTEN DES VAGINALZYTOGRAMMS BEI BEAGLE-HUENDINNEN WAEHREND TOXIKOLOGISCHER LANGZEITUNTERSUCHUNGEN VON GESTAGENEN" ZEITSCHRIFT FUER VERSUCHSTIERKUNDE, GUSTAV FISCHER JENA VERLAG, JENA, DE, Bd. 24, Nr. 3, 1982, Seiten 117-125, XP001022727 ISSN: 0044-3697
- Kuhl, Hoffmann: "Kontrazeption" 1999, Georg Thieme Verlag
- Taubert, Kuhl: "Kontrazeption mit Hormonen" 1995, Georg Thieme Verlag

## Beschreibung

### Technisches Gebiet

Die Erfindung betrifft ein Mehrphasenpräparat zur Kontrazeption auf Basis von Estradiolvalerat mit Dienogest.

Dieses Mehrphasenpräparat realisiert im Vergleich zu den gattungsgemäßen herkömmlichen ovulationshemmenden Präparaten, die sich seit langem in der breiten Anwendung als zuverlässig und sicher erwiesen haben, über die gesamte Dauer des Zyklus eine höhere kontrazeptive Sicherheit, verbessert das zyklische Blutungsverhalten und minimiert bzw. schließt Nebenwirkungen, wie Brustspannen, Kopfschmerzen, depressive Verstimmungen und Libidoveränderungen u.ä. aus.

### Stand der Technik

Aus der Patentliteratur sind Mehrphasenpräparate auf der Basis natürlicher Estrogene in Kombination mit Gestagenen bekannt.

Die Patentschrift EP 0 770 388 B1 beschreibt ein Mehrphasenpräparat zur Kontrazeption, dessen erste Phase aus 2 bis 4 Tagesdosiseinheiten besteht, und jede Tagesdosiseinheit als Wirkstoff ausschließlich natürliche Estrogene enthält. Die zweite Phase des Mehrphasenpräparates besteht aus 2 Gruppen von Tagesdosiseinheiten mit einer Kombination aus mindestens einem natürlichen Estrogen und mindestens einem synthetischen oder natürlichen Gestagen. Dabei wird die erste Gruppe aus 5 bis 3 Tagesdosiseinheiten und die zweite Gruppe aus 17 bis 13 Tagesdosiseinheiten gebildet. Eine dritte Phase besteht aus 2 bis 4 Tagesdosiseinheiten besteht und jede Tagesdosiseinheit enthält als Wirkstoff ausschließlich natürliche Estrogene. Die Tagesdosiseinheit an natürlichem Estrogen bleibt innerhalb der Phasen konstant fällt jedoch von Phase 1 zu Phase 3 ab. Der Anteil an synthetischen oder natürlichen Gestagen übersteigt in der zweiten Gruppe der zweiten Phase den Anteil in der ersten Gruppe. Eine abschließende Phase besteht aus 2 bis 4 Tagesdosiseinheiten und jede Tagesdosiseinheit enthält als Wirkstoff ein pharmazeutisch unbedenkliches Placebo.

Im Anwendungsbeispiel 5 ist eine Kombination von Estradiolvalerat mit Dienogest aufgezeigt. Dabei werden in der ersten Phase 3 Tagesdosiseinheiten zu 3 mg Estradiolvalerat, in der zweiten Phase, in der ersten Gruppe 4 Tagesdosiseinheiten zu 2 mg Estradiolvalerat plus 1 mg Dienogest, in der zweiten Gruppe dieser zweiten Phase 16 Tagesdosiseinheiten zu 2 mg Estradiolvalerat plus 2 mg Dienogest und in der dritten Phase 2 Tagesdosiseinheiten zu 1 mg Estradiolvalerat verabreicht. Die abschließende Phase enthält 3 Tagesdosiseinheiten pharmazeutisch unbedenkliches Placebo.

Für die Aussage zur kontrazeptiven Sicherheit wurde radioimmunologisch die Serumkonzentration von Progesteron gemessen. Es wurde ein Grenzwert von 4,0 ng/ml Progesteron angegeben. Die durchschnittliche Rate der Zwischenblutungen (Durchbruchsblutungen und Spotting) sank um 45 bis 53% vom ersten Einnahmezyklus zum letzten Einnahmezyklus.

Es ist weiterhin bekannt, dass die kontrazeptive Sicherheit von Kombinationspräparaten auf der Wirkung beider Komponenten, des Estrogens und des Gestagens beruht.

Ferner ist auch bekannt, dass die Ovulationshemmdosis für Dienogest täglich 1,0 mg - Dienogest: Präklinik und Klinik eines neuen Gestagens, hrsg. Von A.T.Teichmann, Walter de Gruyter Berlin/New York, (1995), S. 101) und für Drospirenon 2,0-3,0 mg (Rosenbaum P, Schmidt W, Helmerhorst F M et al, Inhibition of ovulation by a novel progestogen (drospirenone), Eur contracept. Reprod. Health Care 5: 16-24 (2000)) bedarf.

Auch zeigen TAUBERT, H.-D. und KUHL, H. (Kontrazeption mit Hormonen, Hrsg. Taubert, H.-D. et al., Georg Thieme Verlag Stuttgart/New York, (1995), S. 160) auf, dass es keinerlei Zusammenhang zwischen dem Auftreten von Zwischenblutungen und niedrigen Serumkonzentrationen des Estrogens, hier Ethinylestradiol, oder des jeweiligen Gestagens gibt.

### Darstellung der Erfindung

Die Erfindung betrifft ein Mehrphasenpräparatat zur Kontrazeption auf Basis eines natürlichen Estrogens
mit einem synthetischen Gestagen, dadurch gekennzeichnet, dass die erste Phase aus 2 Tagesdosiseinheiten
des natürlichen Estrogens Estradiolvalerat zu 3 mg besteht,
eine zweite Phase aus 2 Gruppen von Tagesdosiseinheiten besteht, wobei die
erste Gruppe aus 5 Tagesdosiseinheiten einer Kombination von 2 mg Estradiolvalerat und 2 mg an Dienogest
und die zweite Gruppe aus 17 Tagesdosiseinheiten einer Kombination von 2 mg Estradiolvalerat und 3 mg an Dienogest,
eine dritte Phase aus 2 Tagesdosiseinheiten mit 1 mg Estradiolvalerat besteht,
und eine weitere Phase aus 2 Tagesdosiseinheiten an pharmazeutisch unbedenklichem Placebo besteht.

Die Erfindung betrifft außerdem Mehrphasenpräparatat zur Kontrazeption auf Basis eines natürlichen
Estrogens mit einem synthetischen Gestagen,
dadurch gekennzeichnet, dass die erste Phase aus 2 Tagesdosiseinheiten des natürlichen Estrogens Estradiolvalerat zu 3 mg besteht,
eine zweite Phase aus 2 Gruppen von Tagesdosiseinheiten besteht, wobei die erste Gruppe aus 5 Tagesdosiseinheiten einer Kombination von 2 mg Estradiolvalerat und 3 mg an Dienogest
und die zweite Gruppe aus 17 Tagesdosiseinheiten einer Kombination von 2 mg Estradiolvalerat und 4 mg an Dienogest,
eine dritte Phase aus 2 Tagesdosiseinheiten mit 1 mg Estradiolvalerat besteht,
und eine weitere Phase aus 2 Tagesdosiseinheiten an pharmazeutisch unbedenklichem Placebo besteht.

Das erfindungsgemäße Mehrphasenpräparat ist besonders zur oralen Applikation geeignet, aber
auch intravaginale, parenterale, inklusive topische, rektale, intranasale, intrabukkale oder sublinguale Applikationen sind als Darreichungsformen denkbar.

Das Mehrphasenpräparat wird mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Für die orale Applikation kommen vorzugsweise Tabletten, Filmtabletten, Dragees oder Hartgelatinekapseln zur Anwendung.

### Ausführungsbeispiele

Die Erfindung soll an einigen Anwendungsbeispielen demonstriert werden. Dabei wird insbesondere die kontrazeptive Sicherheit, das zyklischen Blutungsverhaltens der Frau sowie die Verträglichkeit der Applikationsregime nach- gewiesen.

### Verträglichkeit

Die Verträglichkeit von wurde anhand subjektiver Empfindungen, wie Kopfschmerzen, depressive Verstimmungen, Brustspannen, Magenbeschwerden (Übelkeit/Erbrechen), Ödeme und Libidoveränderungen bewertet.

### Anwendungsbeispiel 1

Folgendes Regime kam zur Anwendung:

| | |
|---|---|
| Tage 1 bis 2 | 3 mg Estradiolvalerat/d |
| Tage 3 bis 7 | 2 mg Estradiolvalerat/d + 2 mg Dienogest/d |
| Tage 8 bis 24 | 2 mg Estradiolvalerat/d + 3 mg Dienogest/d |
| Tage 25 bis 26 | 1 mg Estradiolvalerat/d |
| Tage 27 bis 28 | Placebo |

Die Studie wurde an 93 Probandinnen im Alter von 18 bis 35 Jahren durchgeführt. Die Einnahmedauer betrug jeweils 3 Zyklen, wobei nur die Zyklen 2 und 3 beobachtet wurden.

Im 2. Zyklus (Primärzielvariable) ovulierten 3 von 93 Frauen (3,23%), im 3. Zyklus 2 von 92 Frauen.

Damit konnte die sichere Ovulationshemmung von 96,77 % bei Anwendung des erfindungsgemäßen Applikationsregime dokumentiert werden.

Gleichzeitig ist eine gute Verträglichkeit unter Einnahme des erfindungsgemäßen Applikationsregimes zu verzeichnen.

### Anwendungsbeispiel 2

| | |
|---|---|
| Tage 1 bis 2 | 3 mg Estradiolvalerat/d |
| Tage 3 bis 7 | 2 mg Estradiolvalerat/d + 3 mg Dienogest/d |
| Tage 8 bis 24 | 2 mg Estradiolvalerat/d + 4 mg Dienogest/d |
| Tage 25 bis 26 | 1 mg Estradiolvalerat/d |
| Tage 27 bis 28 | Placebo |

Die Studie wurde an 93 Probandinnen im Alter von 18 bis 35 Jahren durchgeführt. Die Einnahmedauer betrug jeweils 3 Zyklen, wobei nur die Zyklen 2 und 3 beobachtet wurden.

Im 2. Zyklus (Primärzielvariable) ovulierten 2 von 93 Frauen (2,15 %), im 3. Zyklus 2 von 92 Frauen.

Damit konnte auch die sichere Ovulationshemmung von 97,85 % bei Anwendung des erfindungsgemäßen Applikationsregime dokumentiert werden.

Gleichzeitig ist eine gute Verträglichkeit unter Einnahme des erfindungsgemäßen Applikationsregimes zu verzeichnen.

Mit beiden Anwendungsbeispielen kann eine ausreichende Ovulationshemmung von 97,85 % bzw. 96,77 % dokumentiert werden. Neueste Untersuchungen mit herkömmlichen Ovulationshemmern nach Pierson R A et al., "Ortho Evra/Evra versus oral contraceptives: follicular development ...", Fertil. Steril. 80 (1), pp. 34-42 (2003) realisieren auch bei Präparaten, die sich seit langem in der breiten Anwendung als zuverlässig und sicher erwiesen haben, in einem gewissen Prozentsatz Ovulationen. Im 2. Behandlungszyklus konnten z.B. bei einem dreiphasigen Levonorgestrel enthaltenden oralen Kontrazeptivum 14 % (3 von 22), bei einem monophasischen Levonorgestrel enthaltenden oralen Kontrazeptivum (6 von 25) und bei einem triphasischen Norgestimat enthaltenden oralen Kontrazeptivum 16% (4 von 25) Ovulationen beobachtet werden. Diese Werte liegen deutlich über denen der erfindungsgemäßen Präparate, so dass bei diesen mit einer höheren Zuverlässigkeit im Vergleich zu Pierson et al. gerechnet werden kann.

## Patentansprüche

1. Mehrphasenpräparat zur Kontrazeption auf Basis eines natürlichen Estrogens mit einem synthetischen Gestagen,
**dadurch gekennzeichnet, dass** die erste Phase aus 2 Tagesdosiseinheiten des natürlichen Estrogens Estradiolvalerat zu 3 mg besteht,
eine zweite Phase aus 2 Gruppen von Tagesdosiseinheiten besteht, wobei die erste Gruppe aus 5 Tagesdosisein- heiten einer Kombination von 2 mg Estradiolvalerat und 2 mg an Dienogest
und die zweite Gruppe aus 17 Tagesdosiseinheiten einer Kombination von 2 mg Estradiolvalerat und 3 mg an Dienogest,
eine dritte Phase aus 2 Tagesdosiseinheiten mit 1 mg Estradiolvalerat besteht,
und eine weitere Phase aus 2 Tagesdosiseinheiten an pharmazeutisch unbedenklichem Placebo besteht.

2. Mehrphasenpräparat zur Kontrazeption auf Basis eines natürlichen Estrogens mit einem synthetischen Gestagen,
**dadurch gekennzeichnet, dass** die erste Phase aus 2 Tagesdosiseinheiten des natürlichen Estrogens Estradiolvalerat zu 3 mg besteht,
eine zweite Phase aus 2 Gruppen von Tagesdosiseinheiten besteht, wobei die erste Gruppe aus 5 Tagesdosiseinheiten einer Kombination von 2 mq Estradiolvalerat und 3 mg an Dienogest
und die zweite Gruppe aus 17 Tagesdosiseinheiten einer Kombination von 2 mq Estradiolvalerat und 4 mg an Dienogest,
eine dritte Phase aus 2 Tagesdosiseinheiten mit 1 mg Estradiolvalerat besteht,
und eine weitere Phase aus 2 Tagesdosiseinheiten an pharmazeutisch unbedenklichem Placebo besteht.

## Claims

1. Multiphase product for contraception based on a natural oestrogen with a synthetic progestogen, **characterized in that** the first phase consists of 2 daily dose units of 3 mg of the natural oestrogen oestradiol valerate,
a second phase consists of 2 groups of daily dose units, where the first group consists of 5 daily dose units of a combination of 2 mg of oestradiol valerate and 2 mg of dienogest
and the second group consists of 17 daily dose units of a combination of 2 mg of oestradiol valerate and 3 mg of dienogest,
a third phase consists of 2 daily dose units with 1 mg of oestradiol valerate,
and a further phase consists of 2 daily dose units of pharmaceutically acceptable placebo.

2. Multiphase product for contraception based on a natural oestrogen with a synthetic progestogen, **characterized in that** the first phase consists of 2 daily dose units of 3 mg of the natural oestrogen oestradiol valerate,
a second phase consists of 2 groups of daily dose units, where the first group consists of 5 daily dose units of a combination of 2 mg of oestradiol valerate and 3 mg of dienogest
and the second group consists of 17 daily dose units of a combination of 2 mg of oestradiol valerate and 4 mg of dienogest,
a third phase consists of 2 daily dose units with 1 mg of oestradiol valerate,
and a further phase consists of 2 daily dose units of pharmaceutically acceptable placebo.

## Revendications

1. Préparation à plusieurs phases destinée à la contraception à base d'un estrogène naturel avec un gestagène synthétique,
**caractérisée en ce que** la première phase est constituée par 2 unités de dosage journalier de l'estrogène naturel valérate d'estradiol à raison de 3 mg,
une deuxième phase est constituée de 2 groupes d'unités de dosage journalier, le premier groupe étant constitué par 5 unités de dosage journalier d'une combinaison de 2 mg de valérate d'estradiol et de 2 mg de Diénogest et le deuxième groupe est constitué par 17 unités de dosage journalier d'une combinaison de 2 mg de valérate d'estradiol et de 3 mg de Diénogest,
une troisième phase est constituée par 2 unités de dosage journalier comprenant 1 mg de valérate d'estradiol,
et une autre phase est constituée par 2 unités de dosage journalier de placebo pharmaceutiquement inoffensif.

2. Préparation à plusieurs phases destinée à la contraception à base d'un estrogène naturel avec un gestagène synthétique,
**caractérisée en ce que** la première phase est constituée par 2 unités de dosage journalier de l'estrogène naturel valérate d'estradiol à raison de 3 mg,
une deuxième phase est constituée de 2 groupes d'unités de dosage journalier, le premier groupe étant constitué par 5 unités de dosage journalier d'une combinaison de 2 mg de valérate d'estradiol et de 3 mg de Diénogest et le deuxième groupe est constitué par 17 unités de dosage journalier d'une combinaison de 2 mg de valérate d'estradiol et de 4 mg de Diénogest,
une troisième phase est constituée par 2 unités de dosage journalier comprenant 1 mg de valérate d'estradiol,
et une autre phase est constituée par 2 unités de dosage journalier de placebo pharmaceutiquement inoffensif.
